# EUROPEAN PATENT APPLICATION

(11) **EP 3 669 815 A1**
(43) Date of publication of application: **24.06.2020**
(21) Application number: 18214523.5
(22) Date of filing: 20.12.2018
(51) Int. Cl.: A61C 7/00, A61C 5/20, G16H 50/50, G16H 20/40, A61C 13/00

(54) **METHOD FOR DIGITALLY PLANNING A RESTORATIVE CARE OF AT LEAST ONE TOOTH**

(71) Applicant: Sirona Dental Systems GmbH, 64625 Bensheim (DE); DENTSPLY SIRONA Inc., York, PA 17401 (US)
(72) Inventor: WILLERS, Ulf, 64342 Seeheim-Jugenheim (DE); SCHNEIDER, Sascha, 64367 Mühltal (DE); MENZEL, Nicolas, 64646 Heppenheim (DE)
(74) Representative: Özer, Alpdeniz

(57) **Abstract**

A method for the digital planning of a restorative care of at least one tooth in the dentition of a patient includes orthodontic tooth movement in the planning of restorative care.

## Description

### Technical Field

The present invention relates to a method for planning a restorative dental treatment for a patient as well as the implementation of a computer program for this method and a machine-readable data carrier on which such a program is stored.

### State of the Art

Currently, orthodontic treatments and restorative care of the teeth are usually performed as medically independent treatments offered on the one hand in an orthodontic practice and on the other hand in a dental practice. Initially in the first orthodontic treatment, the position and orientation of the existing teeth are corrected. Independent therefrom this can be followed by a further treatment via a restorative care, whereby, for example, the shape of the teeth is corrected.

US Patent Application US 2018/0078335 A1 describes a method for the coordination of orthodontic and cosmetic dental treatment, wherein first an orthodontic treatment plan for the correction of a false bite is created. Based on the degree of partial correction of the false bite, a portion of the tooth is determined for cosmetic care and then the tooth is moved accordingly before the cosmetic restorative treatment is performed.

The international patent application document WO 2009/048475 A1 discloses a similar procedure method for the development of a treatment plan for the teeth, simulating orthodontic treatment on the basis of a virtual model of the patient's dentition. A subsequent tooth replacement treatment is also simulated, taking into account a so-called restorative parameter, which can be, for example, the volume of the tooth material to be removed.

In the case of a change in tooth shape, veneering of the teeth is often performed by using so-called veneers. A veneer for the teeth is formed by a very thin, translucent ceramic shell and is applied with special adhesive to the tooth surface, especially in the area of the anterior teeth. For the application of conventional veneers on the teeth, a thin layer up to about 1 mm is usually removed from the enamel before a mold is made for the production of the veneers. The bonding of the ceramic of the veneer with the tooth is usually done with a composite plastic adhesive. Recent techniques in the production of veneers allow the production of veneers with significantly lower material thickness. These so-called non-prep-veneers can be applied without preparing the tooth surface, i.e. directly on the hard substance of the tooth without prior grinding of the tooth. A preparation in the interdental areas is usually not required to apply the veneers.

The invention has the object of improving the restorative care of the teeth, in particular the dental material of the patient should be conserved.

### Description of the Invention

This object is achieved by a method for digitally planning a restoration of at least one tooth in the dentition of the patient, as is the subject of the main claim. Preferred configurations of this method, as well as a computer program for carrying out the method and a machine-readable data carrier result from the further claims.

The inventive method is based on a restorative care of at least one tooth. The key point of the method is that an orthodontic tooth movement is included in the planning of restorative care. By first performing the orthodontic movement of the tooth before the restorative care, it is possible to dispense with a preparation of the teeth, especially in the interdental space largely or completely. Thus, in the planning of the restorative care , an achievable final arrangement of at least one moved tooth in the context of orthodontic tooth movement can be taken into account such that no or only minimal preparation of the tooth or teeth, in particular in the interdental space is required for the restoration of the teeth. Minimal preparation means that less needs to be prepared compared to conventional methods, as a result, less dental material needs to be removed. For orthodontic tooth movement, the advantage arises that the tooth movement for at least one moving tooth is generally low compared to conventional orthodontic treatments and requires relatively little time. The orthodontic tooth movement only has to take place up to the point or up to the movement target until the tooth substance has moved/rotated into the desired end position so far that a restoration can preferably be applied to the tooth substance without preparation, which then produces the desired target situation. This form of orthodontic tooth movement is significantly faster compared to conventional orthodontic treatments because the tooth or teeth need not be moved to a final target. In addition, when compared with conventional orthodontic treatments, the often cumbersome final fine-tuning of the teeth in the final phase of treatment omits.

The restorative care of the teeth is in particular for a tooth shape change. Particularly preferred in this case is a preparation-free veneering of at least one tooth with a veneer. In this case, so called non-prep veneers may be used, in which it is possible to dispense with grinding of the tooth surface and removal of the enamel by means of particularly low material thicknesses. Thanks to the inventive method a preparation of the interdental spaces can also be dispensed or at least largely dispensed with, since the space required for the veneers in the interdental space is provided by the orthodontic tooth movement.

In a preferred embodiment of the method, in the case of orthodontic tooth movement, an end position of the moved tooth or teeth is included in the planning such that minimum material wall thicknesses of the restoration material, for example the non-prep veneers, are taken into account. In this way, a restorative restoration of the teeth can even be carried out without preparation, whereby a preparation, in particular in the region of the interdental spaces, can be dispensed with in order to produce the required space for the minimum wall thicknesses.

The restorative care can be motivated both aesthetically and medically, for example to close tooth gaps.

The digital planning of the restorative care can be carried out, for example, using dental databases or case-specific individually (e.g., using biogeneric tooth models, for example with Biokiefertechnologie (bio jaw technology), Sirona Dental Systems GmbH, Germany). Biogeneric tooth models are based on digitization of individual teeth and, if necessary, whole jaws, whereby mathematical transformations can derive the tooth shape for teeth to be restored. The restorative planning can be automated and case-specific individually or, for example, manually by using tooth databases.

Preferably, the digital planning of restorative care may include functional aspects of the patient's dentition. In particular, the effects of restorative care on adapted joint positions, altered force introduction into the bone, dynamic or static contacts, simulated correct pronunciation/tongue movement, and the like may be considered.

Furthermore, aesthetic aspects of the patient's dentition can be included in the digital planning of the restorative care, such as an adjustment of gum/tooth limits and their impact on the aesthetic appearance, as well as, for example, influences of the restorations on the smile line and/or the harmony of the dentition, also in combination with the face shape and the overall appearance. In particular, a so-called smile design can be included in the digital planning of the restorative restoration in the context of the method according to the invention, with which the planned result of the treatment is visualized to check effects on the smile or laugh of the patient and adjust if necessary.

It is preferably envisaged that the result of digital planning will be visualized. Here, various possible results of digital planning can be visualized in a comparative manner, whereby the various possible results can be simulated with and without tooth movement and with and without restorative care, in particular with and without cosmetic restorative care. For example, the visualization can be a planned result without preparation for restorative care with previous orthodontic tooth movement and/or the result of a restorative care with preparation and previous orthodontic tooth movement and/or include the result of a restorative care of teeth with preparation of the teeth and without previous orthodontic tooth movement. In this way, it is possible to show and visualize all possibilities in advance to the patient and also to the dentist, so that the optimal and most appropriate decision can be made for the patient as to which treatment option should be selected. On the other hand, it is also possible that by the method an end arrangement of the natural teeth after completion of the orthodontic repositioning portion of the treatment is automatically proposed so that no preparation of the natural teeth is necessary and yet the required space for the minimum wall thickness of the veneering material or the restoration material is achieved. By using the described method in a system, it is thus possible to perform an automated planning of a restorative treatment based on a combined treatment of orthodontics and restorative care.

The method described may preferably be carried out as part of a computer-assisted design program and/or a computer-aided manufacturing program for the planning and/or manufacturing of dental and/or dental cosmetic restorations. Such so-called CAD or CAM programs are already being used for the digitized planning of restorative and orthodontic dental treatments (CAD/CAM technology) and can be expanded with the described methods.

The invention further comprises a computer program for carrying out the described method as well as a machine-readable data carrier on which such a computer program is stored.

The invention further comprises a cosmetic method for the restorative care of at least one tooth with a cosmetic veneer. In this case, a pre-treatment of the tooth or teeth is first performed by an orthodontic tooth movement taking into account the planned restorative care. Next, this or the Veneer(s) are applied without preparation or in comparison with conventional methods with reduced preparation effort (glued). For this purpose, non-prep veneers are preferably used.

Additional features and advantages of the invention emerge from the following description of design examples in conjunction with the drawings. The individual features can hereby be realized individually or in combination with one another.

### Brief Description of the Drawings

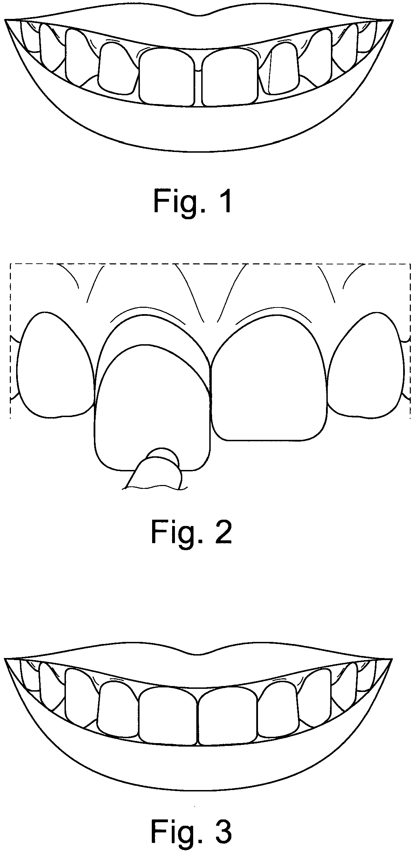

Figures 1 to 3 show three stages of the restorative dental care according to the method of the invention.

### Design Examples

Figures 1 to 3 show the various stages of a restorative care of the teeth, which is digitally planned with the method according to the invention. **Fig. 1** shows the arrangement of the teeth after a successful orthodontic tooth movement, with small interdental spaces are present or were generated by the tooth movement, which are required for a substantially preparation-free attachment of veneers. This tooth set-up is planned digitally in advance with the method according to the invention in order to provide the space required for restorative care. **Fig. 2** shows the attachment of the veneer on the teeth, in particular on the anterior teeth. **Fig. 3** shows the final result with attached veneers. The digital planning of this restorative care within the framework of the inventive process has the particular advantage that, especially in the case of non-prep veneers for the restorative care, no or substantially no grinding of the natural teeth is required because the space required is provided by the orthodontic tooth movement. The planned target of the orthodontic tooth movement takes into account the material wall thicknesses of the restoration material, so that the subsequent restorative care can be provided without preparation.

By visualizing possible treatment plans with and without the involvement of orthodontic tooth movement, the different results can be directly compared with each other before starting treatment. For example, it can be simulated to show what the result looks like with preparation-free restorative care with tooth movement compared to a restorative dental care with preparation and if applicable, without tooth movement. In addition, it can be visualized comparatively what result an orthodontic tooth movement without restorative care may be achieved. By such an increased prognostic security before the start of the treatment, the attending dentist and the patient achieve better predictability and an increased probability of occurrence of the target, since in this way the target can be made completely visible before a start of treatment.

The inventive method can also be used, for example, for the planning of an orthodontic treatment, by providing a final result of treatment without and with restorative final care as a decision-making aid for the selection of the treatment plan for the attending dentist and the patient. In addition, it can also be simulated to show what, in the case of a restoration, the final result will look like when it is done with and without preparation of the repositioned teeth. The calculation of the restorative parts of the converted teeth can be done manually, for example, a virtual manual preparation in the digital model is possible. Alternatively, the calculation of a required preparation can also be automated by taking into account, in particular, the space requirement for minimum wall thicknesses of veneering material. Thus, a manual or an automated variant of preparation planning can be realized on the basis of a planned orthodontic tooth movement or a planned orthodontic treatment.

At the end of the day, even if a chewing situation is completely free to be redesigned, the user could check the result of a combination of orthodontics and restorative care to see whether the planned goal is even achievable or how large the respective treatment shares must be in order to reach the planned target.

The described method is particularly suitable as an extension of a restoration proposal calculation, for example in the context of bio jaw technology, whereby the final arrangements of the teeth attainable by the tooth movement are incorporated in the proposal calculation for the restorations. In addition, the described method can also be used or implemented as an extension for a teeth arrangement planning within the scope of an orthodontic treatment, in that the restorative care is incorporated in the planned end position of the teeth in order to minimize a preparation requirement of the teeth. By automated combination of both techniques, restorative care and orthodontic care, end situations can be calculated automatically or, if necessary, manually planned, which cannot or can only partially be reached in case of a sequential application or a restriction to one of the two treatment methods. Above all, the method described can be used in the course of visualizing the treatment results or the possible variants of treatment results in order to serve as a decision-making aid for the concrete planning of the treatment. By comparing outcomes taking into account potentially different shares of restorative care and orthodontic tooth movement, it may be decided which treatment method or combination of treatment methods is most appropriate for the patient. In addition, the method can also be incorporated directly in the preparation planning.

## Claims

1. Method for the digital planning of a restorative care of at least one tooth in the dentition of a patient, **characterized by** the inclusion of orthodontic tooth movement in the planning of restorative care.

2. Method according to claim 1, **characterized by** the fact that in the planning of restorative, an achievable arrangement of at least one moving tooth is taken into account within the framework of the orthodontic tooth movement in such a way that for the restorative care of the at least one tooth no preparation or a preparation with reduced effort is required compared to conventional methods.

3. Method according to claim 1 or claim 2, **characterized by** the fact that the restorative care of the at least one tooth includes a tooth shape change.

4. Method according to any of the previous claims, **characterized by** the fact that the restorative care of the at least one tooth comprises a veneering without preparation of at least one tooth with a veneer.

5. Method according to any of the previous claims, **characterized by** the fact that with the orthodontic tooth movement a final arrangement of the at least one moving tooth includes the consideration of minimum material wall thicknesses of restorative material.

6. Method according to any of the previous claims, **characterized by** the fact that the digital planning of the restorative care takes place on the basis of dental databases or case-specific individual.

7. Method according to any of the previous claims, **characterized by** the fact that functional aspects of the dentition of the patient are included in the digital planning of the restorative care.

8. Method according to any of the previous claims, **characterized by** the fact that aesthetic aspects of the dentition of the patient are included in the digital planning of the patient's dentition.

9. Method according to any of the previous claims, **characterized by** visualizing the result of digital planning.

10. Method according to any of the previous claims, **characterized by** the comparative visualization of various possible results of digital planning, whereby the various results include a planned restorative care of the at least one tooth without preparation and with orthodontic tooth movement and/or a restorative care of at the least one tooth with preparation and orthodontic tooth movement and/or a planned restorative care of the at least one tooth with preparation and without orthodontic tooth movement.

11. Method according to any of the previous claims, **characterized by** the fact that the method is executed within the framework of a computer-aided design program and/or manufacturing program for the planning and/or manufacture of dental and/or dental cosmetics restorations.

12. Computer program for implementing a method according to any one of Claims 1 to 11.

13. Machine-readable data carrier, on which a computer program according to Claim 12 is stored.

14. Method for restorative care of at least one tooth with a cosmetic veneer, **characterized by** the fact that a pre-treatment of the teeth or an orthodontic tooth movement occurs, taking into account the planned restorative care and the veneer is then applied without preparation or in comparison with conventional methods with reduced preparation effort.
